# EUROPEAN PATENT APPLICATION

(11) **EP 1 762 207 A1**
(43) Date of publication of application: **14.03.2007**
(21) Application number: 05019777.1
(22) Date of filing: 12.09.2005
(51) Int. Cl.: A61F 13/15, A61F 13/58, A61F 13/56, B32B 5/04

(54) **Laminate disposal tape tab and method for manufacturing the same**

(71) Applicant: 3M Innovative Properties Company, St. Paul, Minnesota 55133-3427 (US)
(72) Inventor: Dahm, Axel, 40668, Meerbusch (DE); Tuschy, Joerg, 50181, Bedburg (DE)
(74) Representative: Vossius & Partner

(57) **Abstract**

The present invention relates to a laminate disposal tape tab (2) comprising adjacent first and second tape tab elements (4, 6), each having a first face and a second face. Preferably, the first face of each tape tab element is at least partially coated with a pressure-sensitive adhesive (26, 28). A plastically deformable low yield extendible film (20) having two end portions (22, 24) and an intermediate portion provided therebetween is attached to the first face of each of the first and second tape tab elements. A blanking film (22) is preferably attached to the first face of each of the first and second tape tab elements and interposed between the tape tab elements and the low yield extendible film such that the low yield extendible film is not in contact with the adhesive coating of the tape tab elements at its intermediate portion.

## Description

### Field of the Invention

The present invention relates to a novel laminate disposal tape tab for use in disposable absorbent articles, particularly disposable pants or shorts style infant diapers or adult incontinence articles, and to a method of manufacturing the same.

### Background of the Invention

Disposable pants or shorts style infant diapers or adult incontinence articles of this type are generally comparable to ordinary underwear pants and do not comprise closure or fastening tabs like open style disposable diapers. When such pants style infant diapers have been used and got soiled there is a desire to compact the absorbent article before final disposal. Since the pants style diapers or incontinence articles do not have closure or fastening tabs which could be used to compact the diaper or incontinence article before final disposal a separate so-called disposal tape has been proposed.

For example, EP 0 623 330 A2 (Hayase et al.) discloses a disposable diaper of the shorts type including a liquid permeable topsheet, a liquid impermeable backsheet, and an absorber interposed between the topsheet and the backsheet. Front and rear waist portions are connected together at opposite sides thereof, respectively. The diaper furthermore comprises a tape fastener adapted to fasten the diaper when the diaper is to be discarded. This tape fastener is disposed at a central section of an outer surface of the backsheet and extends in the longitudinal direction of the backsheet. This fastening tape is provided with a single fold or a Z-fold in order to provide a fastening tab of sufficient length to close the diaper in its compacted shape when it is to be discarded.

US 5 807 371 A (Toyoda et al.) discloses an extendible tape comprising a base part and an extendible part linked to the base part. The base part is formed of a material that does not undergo plastic deformation under a load applied in a longitudinal direction of the tape of generally not more than 2000gf (20N)/25mm of width of the tape. The extendible part is formed of a material that undergoes plastic deformation under a load applied in the longitudinal direction of the tape of generally not more than 2000gf (20N)/25mm of width of the tape. The length of the extendible part after stretching is substantially larger than the length of the base part, while the width of the extendible part after stretching is substantially smaller than the width of the base part. The extendible part is extendible under load to sanitarily contain the disposable absorbent article.

EP 0 752 239 A1 (Arakawa et al.) discloses an expandable fixing tape comprising a base material composed of an expandable portion and a substantially non-stretchable portion, and a fixing means provided on at least one non-stretchable portion which is present on at least one end of the base material. The expandable portion exhibits a load (tension) of not more than 5.0 kg/25mm when it is subjected to 200% expansion and a permanent set of not less than 30% under a condition of 200% expansion. The substantially non-stretchable portion has a ductility of less than twice under a load of not more than 0.5 kg/25mm. According to another aspect of EP 0 752 239 A1 the expandable fixing tape comprises a base material composed of an expandable portion, which is a thin layer portion having a thin thickness, at the central portion and the lengthwise direction and substantially non-stretchable portions which are thick layer portions having a thick thickness, at both end portions. The fixing tape furthermore comprises a fixing means provided on at least one non-stretchable portion which is present on at least one end of the base material. The expandable portion can be expanded to the length of at least three times under a load of 1.5 kg/25mm and has a permanent set of at least 30% under the condition of 200% expansion. The thickness of the expandable portion is not more than 90% of the thickness of the non-stretchable portion. The fixing tape has a single fold or a Z-fold.

EP 0 826 352 A2 (Igaue et al.) discloses a disposable securing means used to secure a disposable body fluid absorbent article such as a disposable diaper in a rolled up state for disposal thereof. This securing means comprises an adhesive tape and a release tape. The adhesive tape is fixed at one end portion to the outer surface of the article and has an adhesive zone on the opposite end portion. The release tape extends in alignment with the adhesive tape and has a release portion fixed to the outer surface of the article and a fold-back portion being contiguous to the release portion and folded back adjacent the end of the adhesive tape. The adhesive zone provided on the adhesive tape is releasably bonded to the release portion and the fold-back portion of the release tape is fixed to the inner surface of the free end portion of the adhesive tape.

WO 01/13842 A1 (Matsuda et al.) describes a similar Z-fold construction as the above-mentioned EP 0 826 352 A2. This construction includes a first tape section having a free end portion and a first connection portion opposing the free end portion, a second tape section having a second connection portion and a first anchor portion opposing the second connection portion. The second connection portion is joined to the first connection portion of the first tape section. Furthermore, a third tape section is provided having a third connection portion and a second anchor portion next to the third connection portion. The third connection portion is joined to the second connection portion of the second tape section. The third connection portion is also joined to a branch connection portion between the second connection portion and the first anchor portion of the second tape section.

The above described tape constructions offer an extensibility that is appreciated by consumers since it allows easy wrapping of the soiled disposable product. This extensibility is provided by a zig-zag folded tape (i.e. Z-fold) construction and/or by an elastic zone provided in the tape. A particular disadvantage of these known constructions is their high manufacturing costs that are due to a relatively large material consumption as in Z-fold constructions or governed by the relatively expensive elastic materials.

### Summary of the Invention

It is an object of the present invention to provide an improved disposal tape tab for disposable absorbent articles, in particular disposable pants or shorts style infant diapers or adult incontinence articles, that provides a high level of convenience for the consumer and is more cost attractive than prior art constructions.

The laminate disposal tape tab of the present invention comprises adjacent first and second tape tab elements, each having a first face and a second face. The tab further comprises a plastically deformable extendible film having two end portions and an intermediate portion therebetween. One end portion is attached to the first face of the first tape tab element, and the other end portion is attached to the first face of the second tape tab element. The intermediate portion of the deformable extensible film plastically extends when stretching the disposal tape tab of the present invention in the direction of its length. Since the intermediate portion is not attached to the tab elements, it is freely extensible, and can extend when stretching the tape tab directly.

In accordance with a preferred embodiment of the present invention, the first face of each tape tab element is at least partially coated with a pressure-sensitive adhesive, e.g., for attachment to the disposable absorbent article. It is furthermore preferred that a blanking film is attached to the first face of each of the first and second tape tab elements and interposed between the tape tab elements and the extendible film such that the extendible film is not in contact with the adhesive coating of the tape tab elements at its intermediate portion. With this embodiment the tape tab elements can be completely coated with pressure sensitive adhesive and strip coating of adhesive can be avoided which is preferred from a manufacturing point of view, e.g., to reduce costs

The first and second tape tab elements are preferably initially provided as an elongate tape which is centrally and/or intermittently slit. The blanking film preferably also comprises two separate portions provided by centrally slitting the blanking film, wherein the two separate portions are attached to the first and second tape tab elements, respectively. The slit through the tape and/or the blanking film is typically a straight slit but a wavy slit or differently shaped slit may also be applied.

The disposal tape tab of the present invention can be applied to a disposable article, particularly a pants or shorts style infant diaper or adult incontinence article, by means of the adhesive provided at the tape tab elements. If the disposable article is to be discarded, the user can grasp one end of the disposal tape tab and extend the tab by plastic deformation of the low yield extendible film so that the tab becomes sufficiently stretched to be wrapped around the article, and the wrapped and/or compacted article can be secured in this position and discarded. Accordingly, the present invention offers an economic and practicable solution for the manufacturing of a disposal tape tab which at the same time provides a convenient and reliable closure of a soiled disposable article.

In accordance with a preferred embodiment of the present invention, the disposal tape tab further comprises a release tape by means of which the disposal tape tab can be secured to a disposable article in the form of a Y-bond. Preferably, the release tape has a first face coated with a release coating and an opposite second face coated with an adhesive. The release tape is preferably connected to one of the tape tab elements, particularly to the tape tab element which is not grasped by the user when discarding the tab, by means of a center stripe. While the use of a center strip is preferred, it is also possible to fold back the release tape.

The extendible film preferably is a low yield extendible film. The extendible film used in the disposal tape tab of the present invention preferably has an elastic modulus of more than 3 N/mm², more preferably an elastic modulus in a range of 5 to 100 N/mm², and even more preferably an elastic modulus in a range of 10 to 30 N/mm². Presently preferred materials for the extendible films are polyethylene based low yield films having an elastic modulus of about 8 N/mm² for a 35 µm thick film, about 20 N/mm² for a 50 µm thick film, and about 70 N/mm² for a 100 µm thick film, in both machine direction (MD) and cross machine direction (CD). The measurements of the elastic modulus are performed according to DIN 53371 (tensile testing).

The present invention also relates to a method for manufacturing a laminate disposable tape comprising the steps of providing a substantially inelastic web, slitting the web along its length, and attaching a plastically deformable extendible film to the two web portions on both sides of the slit in order to connect the two web portions. The extendible film preferably is a low yield extendible film. Preferably, one surface of the web is coated with a pressure-sensitive adhesive, and a blanking film is applied onto the pressure-sensitive adhesive along the length of the web in a central portion of the web. Preferably the web, the pressure-sensitive adhesive and the blanking film are slit during a single slitting step. The thus formed laminate tape is then preferably cut across its length to form a plurality of laminate disposal tape tabs.

Preferably, the above method for manufacturing a disposable tape comprises the additional steps of providing a release tape and a center stripe, attaching the release tape and the center stripe to the laminate tape, and rolling up the thus formed laminate to form a laminate roll. Said laminate roll preferably provides a fastening system or disposal system.

A particular advantage of the disposal tape tab of the present invention vis-à-vis- prior art disposal tape tabs lies in that it can be manufactured reliably, precisely and at low costs. The use of a blanking film allows a lamination process to be used and avoids a strip coating process. In particular, it is easier to provide an additional blanking film for partially covering the adhesive layers of the tape tab elements as compared to, e.g., in-line ink coating a very thin layer onto the adhesive.

A further advantage of the disposal tape tab according to the present invention is that less and cheaper materials are required and no complicated connecting or folding processes are required. Furthermore, the disposal tape tab of the present invention can be manufactured as a tape in endless form and converted into a stable roll. Both planetary and level wound roll formats as well as festooning may be used as a format.

Especially advantageous is the construction using a release tape because the use of a release tape results in a better and more secure anchoring of the tape tab on the disposable article, e.g., the diaper. When stretching the tape tab the forces are distributed on the release tape and the tape tab element so that the tape tab element not grasped by the user is less likely to be separated from the disposable article. This is presently contemplated as the best mode for carrying out the invention.

### Brief Description of the Drawings

- Fig. 1: is a perspective schematic view of a pant or shorts style diaper with a disposal tape tab according to the present invention.
- Fig. 2: is a perspective schematic view of a pant or shorts style diaper with a disposal tape tab according to the present invention used to compact the diaper after use.
- Fig. 3: is a schematic side view of an embodiment of a a disposal tape tab of the present invention.
- Fig. 4: is a perspective view of the disposal tape tab illustrated in Fig. 3.
- Fig. 5: is a schematic side view of another embodiment of the disposal tape tab of the present invention with a release tape and a center stripe.
- Fig. 6: is a schematic side view of an alternative embodiment of the disposal tape tab of the invention.
- Fig.7: shows a method for manufacturing the disposal tape tab of the present invention.
- Fig. 8: is a schematic side view of another embodiment of a a disposal tape tab of the present invention.
- Fig. 9: is a schematic side view of still another embodiment of a a disposal tape tab of the present invention.

### Detailed Description of Preferred Embodiments

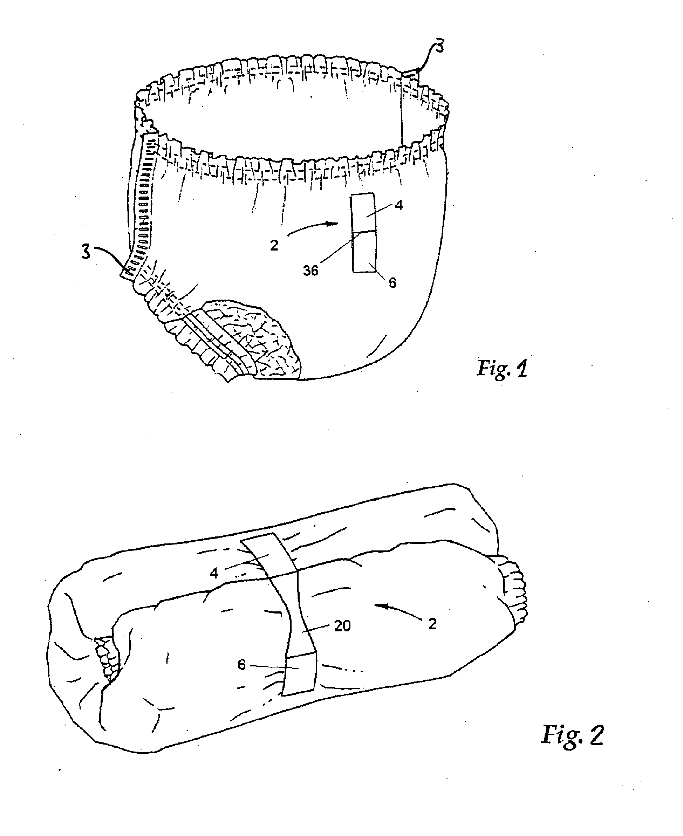

The disposal tape tab 2 of the present invention can be particularly advantageously used in connection with pants or shorts style infant diapers or adult incontinence articles as schematically illustrated in Fig. 1. After use of such a pant style diaper, the pant is typically torn apart along at least one of its seams 3 prior to rolling it up. Removing the soiled diaper over the legs would typically contaminate the legs. Fig. 2 shows a used (soiled) diaper in a rolled-up state after it has been torn along the seams 3. The disposal tape tab 2 is used to hold the soiled diaper in a compacted configuration which typically is a rolled-up configuration as shown in Fig. 2.

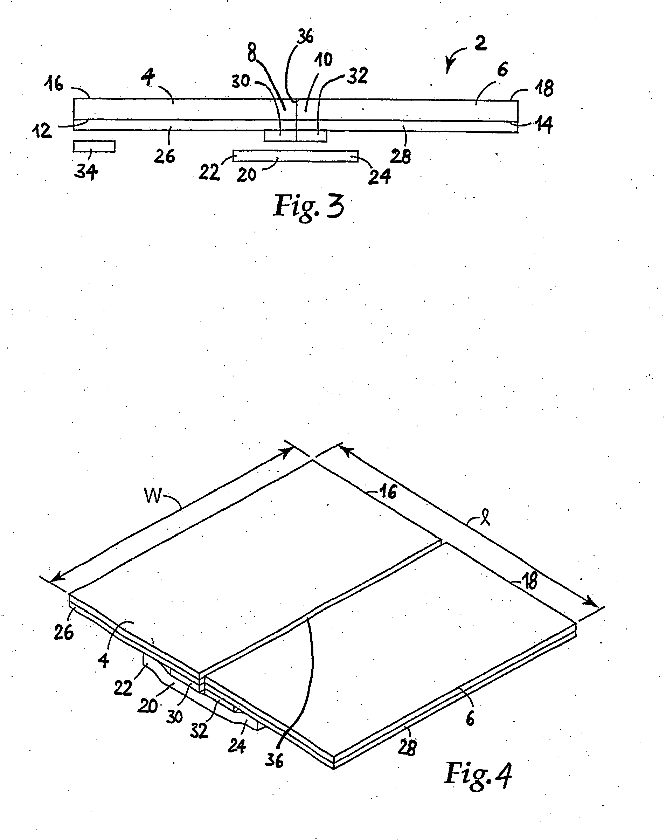

As shown in Figs. 3 and 4, the laminate disposal tape tab 2 of the present invention comprises two adjacent first and second tape tab elements 4, 6 having opposing terminal ends 8, 10. The tape tab elements 4, 6 are preferably made of a non-woven material, or a laminate of a non-woven material and a film layer, or a monolayer film, like a polyolefin film. Each of the tape tab elements 4, 6 has a first face 12, 14 and a second face 16, 18.

A plastically deformable low yield extendible film 20 is shown unattached in Fig. 3 but is in the final tab attached with its two end portions 22, 24 to each tape tab element first face 12, 14. The low yield extendible film 20 preferably has an elastic modulus of more than 3 N/mm², more preferably an elastic modulus in the range of 5 to 100 N/mm², and even more preferably an elastic modulus in the range of 10 to 30 N/mm².

The plastically deformable low yield extendible film may be a monolayer or multilayer film, like a coextruded film. Preferably, the plastically deformable low yield extendible film 20 is attached approximately symmetrically with respect to the opposing terminal ends 8, 10 of the adjacent elements 4, 6, but optionally the film 20 may also be attached asymmetrically. The low yield expandible film 20 can be made from any suitable material and is preferably made from materials selected from the group consisting of PVC, EVA, and PVA. A preferred material is polyolefin, for example, LLDPE (linear low density polyethylene). Materials having a permanent set of at least 50% and more preferably at least 70% are preferred.

The first face 12, 14 of both tape tab elements 4, 6 is preferably completely coated with a pressure-sensitive adhesive 26, 28. The low yield extendible film 20 has its first end portion 22 and its second end portion 24 adhesively connected to the first and second tape tab elements 4, 6, respectively. The film 20 may be attached to the tape tab elements by way of the adhesive 26, 28. Alternatively, or in addition, the film 20 may be bonded to the tape tab elements 4, 6 by means of a thermal bond, an ultrasonic bond or a cold pressure bonding. An intermediate portion of the low yield extendible film 20 between the end portions 22, 24 is inhibited from being adhesively connected to the tape tab elements 4, 6 by means of an interposed blanking film comprising two portions 30, 32 which is provided on the tape tab elements 4, 6 near their terminal ends 8, 10. In the embodiments shown in Figs. 3-5, the overall length of the blanking film 30, 32 is smaller than the length of the low yield extendible film 20 so as to allow direct attachment of the end portions 22, 24 of the extendible film 20 to the tape tab elements 4, 6. The blanking film preferably is a separate film that is laminated to the tape tab elements 4, 6 during manufacturing of the laminate disposal tape tab 2. The blanking film may be made of a transparent or opaque material and can also be colored. The blanking film may be made of a non-woven with an optional laminated film. The length of the blanking film 30, 32, or the distance between the attachment sites of the low yield extendible film 20 to the two tape tab elements 4,6 generally determines the width of the plastically deformable zone or possible elongation of the tape tab.

At a free end of one of the tape tab elements 4, 6 a finger lift 34 is provided which is shown unattached in Fig. 3 and is laminated during the manufacturing process. A slit 36 is provided, preferably centrally, and separates the two tape tab elements 4, 6 and the two portions 30, 32 of the blanking film.

The disposal tape tab 2 according to the present invention is attached to the absorbent article, e.g., the pants or shorts style infant diaper or adult incontinence article (Fig. 1), by means of the adhesive 28. As such, the second tape tab element 6 of the disposal tape tab 2 is a so-called "manufacturer's end" which is non-releasably secured to the diaper (as opposed to a so-called "user's end"). The first tape tab element 4 is used to close a wrapped or compacted soiled diaper after use in order to be discarded. To this end, the adhesive 26 of the first tape tab element 4 may be arrange on a release coating provided on the diaper or may comprise a release liner. After use of the diaper, i.e. when it is to be discarded, the first tape tab element 4 can be used to secure the diaper in its compacted condition as shown in Fig. 2. The first tape tab element 4 is therefore also called a "user's end".

The tape tab elements may comprise a carrier film, which can be a polymer layer or a non-woven layer, wherein a polymer layer or film may be attached to the non-woven layer. The thickness of the polymer film carrier is typically 30 to 120 µm, preferably 40 to 110 µm, more preferably 70 to 110 µm, and even more preferably 95 to 105 µm. The non-woven carrier, which is used with or without a film layer, typically has a basis weight of 25 to 100 g/m2, preferably 30 to 90 g/m2. The thickness of the adhesive 26, 28 is typically in the range of between 10 and 40 µm, preferably about 20 to 35 µm. The thickness of the interposed blanking film 30, 32 is typically 5 to 40 mm, preferably 8 to 20 µm, and even more preferably about 10 to 15 µm. The plastically deformable low yield extendible film 20 preferably has a thickness in the range of between 30 to 100 µm, preferably between 40 and 75 µm and most preferably about 50 µm. The thickness of the tape tab elements is essentially the sum of its individual layers. The various layers of the disposal tape tab are all of such a thickness that the thickness variation of the whole disposal tape in the direction of its length 1 across the tab 2, including the blanking film (if present) and the extendible film, is preferably less than about 50%, and more preferably less than about 40%.

The width W of the disposal tab 2 (see Fig. 4) may vary depending on the size of the disposable article and is generally in the range of between 5 to 50 mm, preferably between 10 and 30 mm, and most preferably between 15 and 25 mm. In the direction of their length 1 (see Figs. 3 and 4) the various elements of the disposal tape tab of the present invention may have the following dimensions: The two tape tab elements 4, 6 measured together may have a length of about 50 to 100 mm, preferably 70 to 90 mm and most preferred about 80 mm. The two portions 30, 32 of the blanking film together may have a length of about 10 to 50 mm, preferably 20 to 40 mm and most preferred about 30 mm, wherein preferably both portions have the same length. The low yield extendible film 20 may have a total length of about 20 to 80 mm, preferably 30 to 50 mm and most preferred about 40 mm, and is preferably applied centrally of the line or slit 36 separating the two tape tab elements 4, 6 and their opposing terminal ends 8, 10.

The finger lift element 34 may have a length of about 5 to 15 mm, preferably 8 to 12 mm and most preferred about 10 mm.

The two adjacent tape tab elements 4, 6 at their opposing terminal ends 8, 10 are preferably in an abutting face to face relation such that they cover substantially the whole low yield extendible film 20. Thus, the film 20 is covered prior to use and is protected from adhesive contamination while also minimizing thickness variation across the disposal tape tab 2 in the central region of the tab. This arrangement has also functional and esthetic advantages. The low yield extendible film 20 can be colored differently and the tape tab elements 4,6 may be provided with designs so as to provide distinguishable contrasts. When the user extends the tab 2 by a certain distance the previously covered low yield extendible film 20 is then clearly exposed, in particular when the blanking film 30, 32 is transparent. This provides a clear visual indication of the extent to which the low yield extendible film 20 is plastically deformed which is also a direct indication of the force being applied. This may be used to indicate a maximum stretchability of the film so that overstretching of the film can be avoided.

Similarly, the opposing ends of the adhesive layers 26, 28 and blanking films 30, 32 are in face to face relation such that they cover substantially all of the intermediate region of the film 20 prior to use.

Typically, the two tape tab elements 4, 6 of the disposal tape tab 2 of the present invention are manufactured from a single continuous web material that is completely covered by an adhesive layer. During the manufacturing process a film ultimately forming the blanking film is then centrally applied onto the adhesive coating of the web. The web material, the adhesive layer and the blanking film are then centrally slit or cut to form the two separate tape tab elements 4, 6 each covered with adhesive layers 26, 28, respectively, and the separate blanking films 30, 32 of appropriately equal lengths. The cut line 36 is shown in Figs. 3 and 4. Subsequently, the low yield extendible film 20 in the form of a continuous tape of sheet is adhesively connected at its lateral end portions 22, 24 to the respective adhesive layer 26, 28. The blanking film is thus completely covered and prevents an adhesive connection between the intermediate portion of the film 20 and the tape tab elements 4, 6. The thus manufactured laminate tape can be wound in a continuous roll. The continuous roll of disposal tape can then be cut into individual disposal tape tabs 2 as illustrated in Fig. 4. These disposal tape tabs 2 can be stretched in the direction of their length 1 due to plastic deformation of the low yield extendible film 20. Upon stretching, a gap between tape tab elements 4 and 6 forms and widens, thus providing an increase length of tape for the closing of the soiled disposable article.

The tape tab elements 4,6 are preferably a laminate of a nonwoven and a film layer but can also be a single film, preferably a polyolefin film, like a polypropylene film or a polyethylene film, or a nonwoven without a film layer. The nonwoven provides softness while a film layer provides a barrier for the adhesive and/or dimensional stability. The nonwoven is preferably a spunbond nonwoven having a basis weight from 10 g/m² to 60 g/m², and the film layer can be extrusion laminated to the spunbond nonwoven. If a laminate of nonwoven and film layer is used, the film layer is preferably adjacent the pressure-sensitive adhesive layer 26, 28. The film layer can also be separately provided and adhesively laminated, thermobonded, sonically bonded and/or otherwise conventionally attached to the nonwoven layer. The nonwoven layer preferably exhibits a release coating on the second face the of elements 4,6 so that the disposal tape can be wound into a roll. The pressure-sensitive adhesive layers 26, 28 are provided on the tape tab elements 4, 6 to both attach the low yield extendible film 20 to the tape tab elements 4,6 and further to attach the tape tab elements 4, 6 to the disposable article. Preferably, both tape tab elements 4,6 have an area beyond the film 20 where the pressure-sensitive adhesive layers 26, 28 are exposed and that can be used to attach the tape tab elements 4,6 to the disposable article or alternatively could be used to attach other elements such as mechanical fastener elements. The end portions 22, 24 of the low yield extendible film 20 preferably extend 2 to 10 mm and preferably about 5 mm over the blanking film portions 30, 32 in both directions. This provides for secure adhesion of the film 20 to the adhesive layers 26, 28 without excessive use of film material. The intermediate region of the low yield extendible film 20 generally corresponds to the total length of the blanking film portions 30, 32.

The low yield extendible film 20 should preferably be capable of being plastically deformed, i.e. stretched, by at least 50%, preferably at least 100% and most preferably at least 150%.

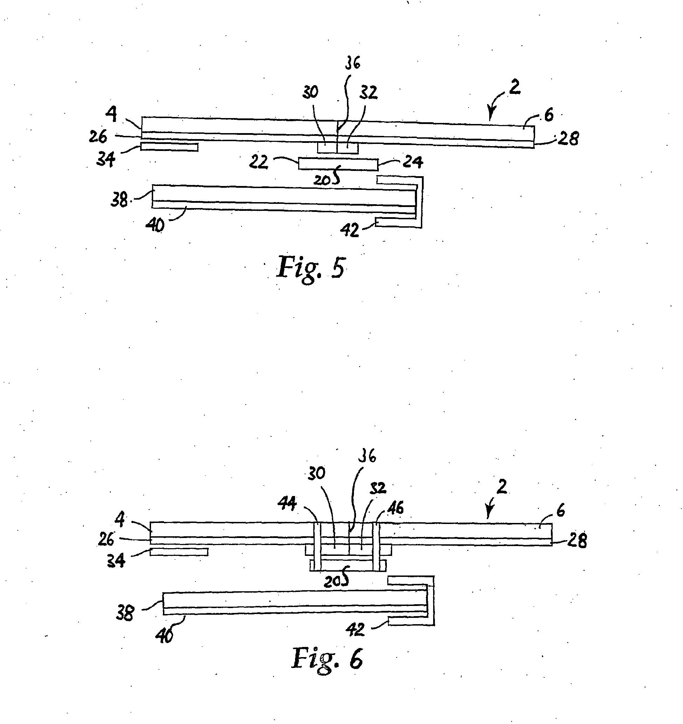

Fig. 5 illustrates a further embodiment of a disposal tape tab 2 of the present invention which in its basic construction generally corresponds to the embodiment shown in Figs. 3 and 4 but additionally comprises a release tape 38. The release tape 38 is particularly useful for attaching the tape tab 2 to the disposable articles so as to provide a so-called Y-bond. This presently preferred embodiment of the invention is particularly advantageous because the use of a release tape results in a better and more secure anchoring of the tape tab on the disposable article. When stretching the tape tab the forces are distributed on the release tape and the tape tab element so that the tape tab element 6 is less likely to be separated from the disposable article.

The release tape 38 is preferably provided with an adhesive layer 40. The release tape 38 is attached to the disposal tape tab 2 at the second tape tab element 6 by means of a center stripe 42. The center stripe is shown for convenience as U-shaped. However, it may rather comprises only one fold, i.e. be V-shaped. Any shape of the center stripe which is appropriate to connect tape tab 2 and center stripe 42 can be used in accordance with the present invention. Preferred materials for the center stripe 42 are, for example, polyester and BOPP (=biaxially oriented PP). While the use of a center stripe is preferred, it is also possible to use a release tape which is folded back

The release tape 38 is preferably provided with a release coating on the side opposite the adhesive layer 40 so that the adhesive 26 provided at the first tape tab element 4 may be readily released from the release tape 38 during use.

The dimensions of the release tape 38 preferably are as follows. The release tape 38 typically has a length of about 40 to 80 mm, preferably about 50 to 70 mm, and even more preferred about 60 mm. The free end of the release tape 38 which is arranged adjacent the finger lift element 34 is preferably spaced at least 2 mm, preferably about 5 mm from the edge of the disposal tape tab 2. The distance between the outer edge of the tape tab element 6 and the release tape 38 is preferably about 5 to 25 mm, more preferably 10 to 20 mm and even more preferred about 15 mm.

Figure 6 shows an alternative embodiment of the disposal tape tab 2 of the present invention. In this embodiment, the blanking film 30, 32 is extended so that its total length is larger than the length of the low yield extendible film 20. In this embodiment the film 20 can be fixed to the extended blanking film 30, 32 by means of thermobonding, ultrasonic bonding or cold pressure bonding. The blanking film 30, 32 is preferable adhesively attached to the tape tab elements 4, 6 by means of the adhesive 26, 28. Additional bonding means, like thermobonding, ultrasonic bonding or cold pressure bonding may be used. Preferably, the low yield extendible film 20 and the two portions 30, 32 of the blanking film are attached to the tape tab elements 4, 6 in a single bonding step by means of thermobonding, ultrasonic bonding or cold pressure bonding, as indicated by reference signs 44, 46 in Fig. 6. In this embodiment the width of the plastically deformable and stretchable zone of film 20 is essentially determined by the distance of the two bonding lines 44, 46 where the film 20 is bonded to the tape tab elements 4, 6.

The dimensions of the disposal tape tab 2 of the embodiment shown in Fig. 6 are essentially the same as described above in connection with Figs. 3 to 5.

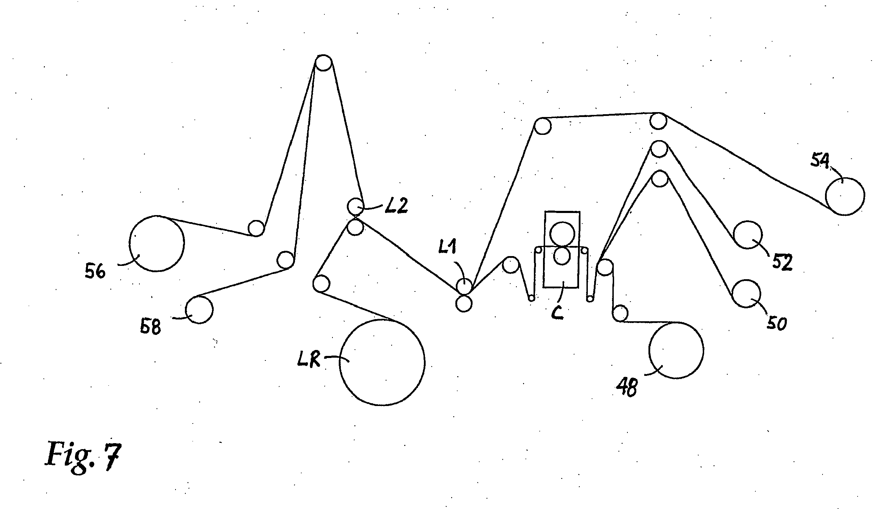

Fig. 7 schematically illustrates a method of manufacturing a disposal tape tab 2 according to the present invention. Fig. 7 is a so-called single laminate roll process.

In the method shown in Fig. 7, material 48 ultimately forming the first and second tape tab elements 4, 6, material 50 ultimately forming the two portions 30, 32 of the blanking film, and material 52 forming the finger lift 34 of the disposal tape tab 2 are provided in endless form from respective rolls via guiding rolls to a cutting station including a cutting knife C where they are slit along their lengths, and further to a first lamination station L1 where they are laminated to the plastically deformable low yield extendible film material 54 also provided in roll form via guide rolls. As discussed above, lamination can be carried out by adhesive bonding, but thermobonding, ultrasonic bonding or cold pressure bonding can be used supplementary or alternatively, in particular in the embodiment where the low yield extendible film 20 is shorter than the blanking film 30, 32. Alternatively, materials 48, 50 can also be cut to form tape tab elements 4, 6 and blanking film portions 30, 32 subsequently to being laminated to the film 54. A partial cutting, or providing a perforation line instead of fully cutting or slitting materials 48, 50 is also an option.

The laminate including, still in the form of an endless sheet or web, the tape tab elements 4, 6, the portions 30, 32 of the blanking film and the low yield extendible film 20 is then provided to a second lamination station L2. In the second lamination station L2 a release tape material 56 and center stripe material 58 are attached to the laminate provided from lamination station L1. Afterwards, the laminate is rolled up on a laminate roll LR. The laminate in roll form can then be provided, e.g., to an in-line diaper production machinery, where individual tape tabs can be cut from said roll.

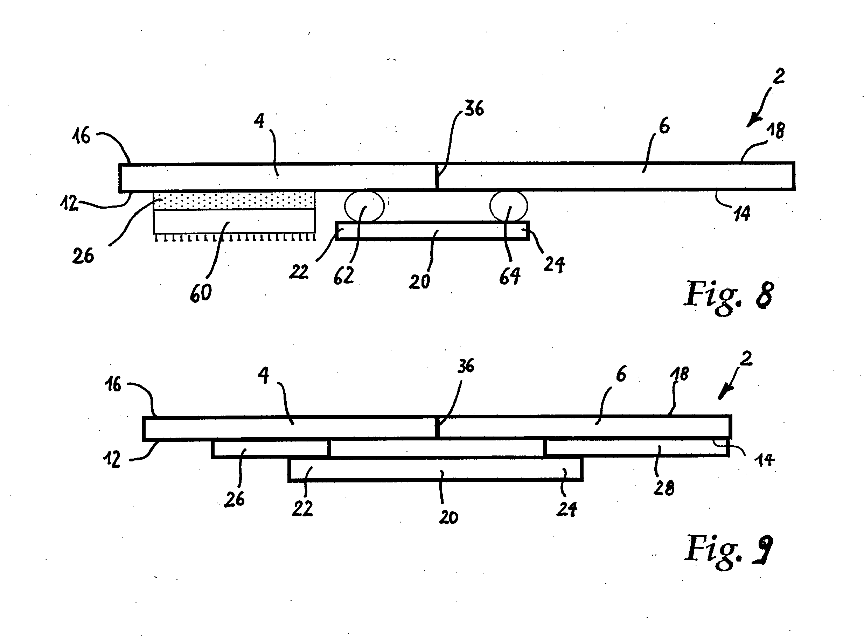

Another embodiment of the disposal tape tab 2 according to the present invention is shown in Fig. 8. As in the previously described embodiments, the disposal tape tab 2 comprises first and second tape tab elements 4, 6 and a low yield extendible film 20. In this embodiment, the first surface 12, 14 of the tape tab elements 4, 6 is not completely covered by an adhesive coating so that no blanking film need to be present. An adhesive layer 26 may be strip coated onto the first face 12 of the first tape tab element 4, i.e. the user's end, by means of which a mechanical fastener element 60 can be attached to the first tape tab element 4. Alternatively, the backside of the mechanical fastener element 60 may exhibit an adhesive layer. A preferred mechanical fastener element is a hook patch 60 since the hooks thereof may readily engage with an exposed non-woven layer of the back sheet of the diaper.

The first face 14 of the second tape tab element 6, i.e. the manufacturer's end, may or may not comprise an adhesive. Although it is preferred that the disposal tape tab 2 is attached to the diaper via an adhesive provided on the first face 14 of the second tape tab element 6, it is also possible to use other bonding mechanisms, such as thermobonding, or ultrasonic welding. If an adhesive is used on the manufacturer's end, a pressure sensitive adhesive or a construction adhesive could be used.

The extendible film 20 may be attached to the first and second tape tab elements 4, 6 by means of adhesive or, as shown in Fig. 8, via bond lines 62, 64 that can be made by thermobonding, ultrasonic bonding and/or any other bonding techniques.

In the embodiment shown in Fig. 8, a release tape 38 may also be attached to the first surface 14 of the second tape tab element 6 in order to provide a Y-bond for attachment of the tab 2 to the diaper, similar to the embodiments shown in Figs. 5 and 6.

In Fig. 9 a further embodiment of the disposal tape tab 2 of the present invention is illustrated. In this embodiment, the first face 12, 14 of the first and second tape tab elements 4, 6 is strip coated with an adhesive, preferably a pressure sensitive adhesive, in order to provide adhesive layers 26, 28. The adhesive layer 26 on the first tape tab element 4 is provided in a center region of the first face 12, and the adhesive layer 28 on the first face 14 of the second tape tab element 6 is provided in a region shifted from the center towards its edge. With this construction an intermediate portion is present between the adhesive layers 26, 28 which is free of adhesive. This intermediate region is bridged by the extendible film 20. The extendible film 20 is attached to the first and second tape tab elements 4 ,6 by means of a partial overlap of the end portions 22, 24 with the adhesive layers 26, 28, respectively. Accordingly, with this embodiment no blanking film is required.

Since the first adhesive layer 26 is strip coated onto the first face 12 in the center region of the first tape tab element 4 only, a finger lift portion without adhesive is provided at the free end of the tab 2. The disposal tape tab 2 can be attached to the disposable article with the non-overlapping part of the adhesive layer 28, i.e. the exposed adhesive layer. A release tape, like release tape 38, may also optionally be used for the attachment. If no release tape is provided it may be necessary to cover the exposed part of the adhesive layer 26 with a release liner or to provide a release coating on the back sheet of the disposable article so that the user's end 4 of the disposal tape tab 2 can be readily pulled from the back sheet in use to bring the disposable article in a compacted state as shown in Fig. 2.

## Claims

1. A laminate disposal tape tab comprising adjacent first and second tape tab elements, each having a first face and a second face, the tape tab further comprising a plastically deformable extendible film having two end portions and an intermediate portion therebetween, one end portion being attached to the first face of the first tape tab element and the other end portion being attached to the first face of the second tape tab element.

2. The tab of claim 1, wherein the first face of each tape tab element is at least partially coated with a pressure-sensitive adhesive.

3. The tab of claim 2, wherein a blanking film is attached to the first face of each of the first and second tape tab elements and interposed between the tape tab elements and the extendible film such that the extendible film is not in contact with the adhesive coating of the tape tab elements at its intermediate portion.

4. The tab of any of claims 1 to 3, wherein the extendible film is attached to the tape tab elements by means of adhesive bonding, thermobonding, ultrasonic bonding and/or cold pressure bonding.

5. The tab of claim 4, wherein the extendible film is attached to the tape tab elements by adhesive bonding and supplementary bonding by means of thermobonding, ultrasonic or cold pressure bonding.

6. The tab of any of claims 1 to 5, wherein the extendible film has an elastic modulus of more than 3 N/mm², preferably an elastic modulus in the range of 5 to 100 N/mm², more preferably an elastic modulus in the range of 10 to 30 N/mm².

7. The tab of any of claims 3 to 6, wherein the blanking film comprises two separate portions being attached to the first and second tape tab elements, respectively.

8. The tab of any of claims 3 to 7, wherein the total length of the blanking film is smaller than the length of the extendible film and the extendible film is directly attached to the first and second tape tab elements at its two end portions.

9. The tab of any of claims 3 to 7, wherein the total length of the blanking film is larger than the length of the extendible film and the extendible film is attached to the first and second tape tab elements via the blanking film.

10. The tab of any of claims 3 to 9, wherein the blanking film is a polyethylene, polyolefm, polyethylene terephtalate, or biaxially oriented polypropylene film, paper, or a nonwoven with or without laminated film.

11. The tab of any of claims 1 to 10, wherein the tape tab elements are made of a monolayer film, e.g., a polyolefin film, a nonwoven, or a laminate of a nonwoven and a film layer.

12. The tab of any of claims 2 to 11, wherein the pressure-sensitive adhesive is exposed on at least one tape tab element.

13. The tab of any of claims 1 to 12, wherein at least one of the tape tab elements is provided with a mechanical fastening element.

14. The tab of any of claims 1 to 13, further comprising a release tape.

15. The tab of claim 14, wherein the release tape has a release coated first face and an adhesive coated second face.

16. The tab of claim 14 or 15, wherein the release tape is connected to one of the tape tab elements by means of a center stripe.

17. A tape comprising a plurality of the tabs of any of claims 1 to 16 arranged side-by-side, wherein the tape is wound into a roll.

18. A pant type diaper comprising a topsheet, a backsheet, an absorbent core and at least one disposal tape tab of any of claims 1 to 16 attached to the backsheet.

19. A method of manufacturing a laminate disposal tape comprising the steps of:
i) providing a substantially inelastic web;
ii) slitting the web along its length to form two web portions; and
iii) attaching a plastically deformable extendible film to the two web portions on both sides of said slit to connect the two web portions.

20. A method of manufacturing a laminate disposal tape tab, comprising the step of cutting the tape manufactured with the method of claim 19 across its length.
